(19) Europäisches Patentamt
European Patent Office
Office européen des brevets

(11) Veröffentlichungsnummer: **0 039 311**
**B1**

(12) # EUROPÄISCHE PATENTSCHRIFT

(45) Veröffentlichungstag der Patentschrift: **11.07.84**

(51) Int. Cl.³: **C 07 J 7/00**, A 61 K 31/57 // C07J13/00, C07J71/00, C12P33/08

(21) Anmeldenummer: **81810152.9**

(22) Anmeldetag: **23.04.81**

(54) **Halogenierte Steroide.**

(30) Priorität: **29.04.80 CH 3303/80**

(43) Veröffentlichungstag der Anmeldung:
**04.11.81 Patentblatt 81/44**

(45) Bekanntmachung des Hinweises auf die Patenterteilung:
**11.07.84 Patentblatt 84/28**

(84) Benannte Vertragsstaaten:
**CH DE FR GB LI NL**

(56) Entgegenhaltungen:
**DE - A - 1 468 171**
**DE - B - 1 113 453**
**GB - A - 898 292**
**GB - A - 1 150 955**
**US - A - 3 557 158**
**US - A - 3 758 524**

**CHEMICAL ABSTRACTS, Band 66, Nr. 9, 26-02-1967, Seite 3650, Zusammenfassung 38117u, Columbus, Ohio, US M. HALMOS et al.: "Steroids. V. Epoxidation of pregnane derivatives and examination of the epoxides"**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Wieland, Peter, Dr., Benkenstrasse 45, CH-4104 Oberwil (CH)**

**EP 0 039 311 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft neue halogenierte Steroide der Formel

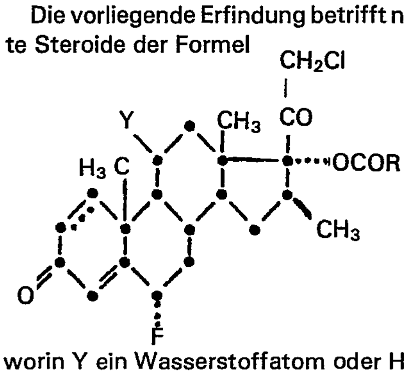

(A)

worin Y ein Wasserstoffatom oder Hydroxyl, und R ein Alkyl mit höchstens 6 Kohlenstoffatomen bedeutet, wobei die punktierte Linie in der 1,2-Stellung eine zusätzliche Doppelbindung eines 1,2-Dehydroderivats bezeichnet, und pharmazeutische Präparate enthaltend diese Steroide, sowie ein neuartiges Verfahren zur Herstellung derselben.

Das durch das Symbol R charakterisierte Alkyl kann verzweigt sein, wie 2-Propyl oder 1,1-Dimethyläthyl, vorzugsweise ist es aber ein geradkettiges Alkyl, wie Methyl, Propyl, Butyl, Pentyl oder Hexyl und vor allem Äthyl.

Zur Herstellung solcher komplizierten polysubstituierten Verbindungen aus einfachen Rohstoffen oder Zwischenprodukten der industriellen Produktion der Steroide benötigt man einen vielstufigen Syntheseweg, in welchem jede einzelne funktionelle Gruppe separat eingeführt wird, wobei oft eine bereits eingeführte Gruppe später gegen eine unerwünschte weitere Umwandlung vorübergehend geschützt werden muss. Die Reihenfolge der einzelnen Syntheseschritte, die meistens als Analogieverfahren an sich bekannt sind, ist oft für die Ökonomie der gesamten Synthese von entscheidender Bedeutung. Bei der Synthesestrategie, d.h. bei der Auswahl von Verfahrensvarianten und ihrer Reihenfolge, wird im allgemeinen besonders geachtet, dass man empfindliche funktionelle Gruppen erst in späteren Stadien der Synthese einführt, um ihre eventuelle Beeinträchtigung durch die nachfolgenden Operationen zu vermeiden. Zu solchen empfindlichen Gruppen wird das 21-Fluor und vor allem das 21-Chlor zweifellos gezählt, zumal wenn seine Reaktivität noch durch eine benachbarte 20-Oxogruppe zusätzlich gesteigert wird. Zur gewöhnlichen Synthesestrategie gehört deshalb allgemein, für die Einführung dieser Halogenatome in die 21-Stellung erst die spätesten Stufen, wenn nicht die allerletzte Stufe, der Synthese vorzusehen. Für die Einführung selbst stehen mehrere, meistens indirekte, Methoden zur Verfügung, denen ein gemeinsames Prinzip zugrunde liegt, nämlich der Austausch einer geeignet veresterten 21-Hydroxylgruppe gegen das gewünschte Halogen, vgl. U.S. Pat. 4 113 680 und U.S. Pat. 3 992 422, sowie den dort angegebenen Stand der Technik. Dafür muss aber die 21-Hydroxylgruppe ihrerseits auch vorher eingeführt werden, wozu im Normalfall wieder mehrere Reaktionsstufen notwendig sind; zudem ist sie selber dermassen reaktiv, dass sie während der Synthese in einer geschützten Form, üblicherweise als Ester, vorliegen muss. Eine solche klassische Synthesestrategie kann man am Beispiel der Synthese von 6α,21-Difluor-11β,17α-dihydroxy-16α-methyl-pregna-1,4-dien-3,20-dion gemäss U.S. Pat. 3 499 016 demonstrieren, worin zur Einführung von 3 funktionellen Gruppen (6α- und 21-Fluor, 17α-Hydroxyl) nicht weniger als 10 Verfahrensstufen notwendig sind, wobei eine sauerstoffhaltige funktionelle Gruppe in der 21-Stellung bereits vom Anfang an vorhanden war.

Dem erfindungsgemässen Verfahren liegt die unerwartete Entdeckung zugrunde, dass 21-Chlor-20-oxosteroide über eine wesentlich höhere chemische Stabilität verfügen, als man bisher allgemein vermutet hat. Wie sich nun überraschenderweise gezeigt hat, bleibt das Chloratom unter normalen Bedingungen verschiedener konventioneller Umwandlungen der technischen Steroidsynthese unversehrt und benötigt zu seiner Erhaltung keine aussergewöhnlichen Vorsichtsmassnahmen, welche den Rahmen üblicher Bedingungen der Produktion überschreiten würde.

Eine konsequente Anwendung dieser Befunde führte zur Erarbeitung des vorliegenden erfindungsgemässen Verfahrens, welches über neuartige Zwischenprodukte auf einem kurzen, übersichtlichen Weg die Verbindungen der Formel A liefert, wie es durch die Formelschemen I und II illustriert wird. In diesen Schemen hat R die oben angegebene Bedeutung und Ac stellt einen Niederalkanoylrest mit 1-7 Kohlenstoffatomen dar, z.B. einen solchen, der sich vom oben definierten Alkylrest R ableitet, besonders aber den Acetyl- und Formylrest. (Zur Unterscheidung davon wird in der ganzen Beschreibung der ähnliche Rest R.CO-, welcher jedoch gemäss der eingangs angegebenen Definition 2-7 Kohlenstoffatome enthält, als der Niederalkancarbonylrest bezeichnet).

Zum Gegenstand der vorliegenden Erfindung gehören auch die neuen Zwischenprodukte zur Herstellung der Verbindungen der Formel A, namentlich das 21-Chlor-3β-hydroxy-16-methyl-pregna-5,16-dien-20-on und Niederalkanoylester davon, das 21-Chlor-3β-hydroxy-16β-methyl-5α,6;16α,17-diepoxy-pregnan-20-on und seine Niederalkanoylester, das 21-Chlor-6β-fluor-3β,5α,17α-trihydroxy-16-methylen-pregnan-20-on und seine 3-Niederalkanoylester und 3-Niederalkanoyl-5,17-diniederalkancarbonylester, das 21-Chlor-6β-fluor-3β,5α,17α-trihydroxy-16β-methyl-pregnan-20-on und seine 5,17-Diniederalkancarbonylester und 3-Niederalkanoyl-5,17-diniederalkancarbonylester, das 21-Chlor-6β-fluor-5α, 17α-diniederalkancarbonyloxy-16β-methyl-pregnan-3,20-dion und das 21-Chlor-6β-fluor-17α-niederalkancarbonyloxy-16β-methyl-pregn-4-en-3,20-dion. Ebenfalls gehört zum Gegenstand der Erfindung das Verfahren zu ihrer Herstellung gemäss Schema I. bzw. II.

Besonders bevorzugte Verbindungen sind dabei: 21-Chlor-3β-hydroxy-16-methyl-pregna-5,16-dien-20-on-formiat und -acetat, 21-Chlor-3-hydroxy-16β-methyl-5α,6;16α,17-diepoxy-pregnan-20-on-formiat und -acetat, 21-Chlor-6α-fluor-3β,5α,17α-trihydroxy-16-methylen-pregnan-20-on-3-formiat und 3-acetat, sowie 3-formiat-5,17-dipropionat und -3-acetat-5,17-dipropionat, 21-Chlor-6β-fluor-3β,-

5α,17α-trihydroxy-16β-methyl-pregnan-20-on-5,-16-dipropionat, -3-formiat-5,17-dipropionat und -3--acetat-5,17-dipropionat, 21-Chlor-5α,17α-dihydroxy-16β-methyl-pregnan-3,20-dion-5,17-dipropionat, 21-Chlor-6α-fluor-17α-hydroxy-16β-methyl--pregn-4-en-3,20-dion-17-propionat, 21-Chlor-6α--fluor-17α-hydroxy-16β-methyl-pregna-1,4-dien--3,20-dion-17-propionat, sowie 21-Chlor-6α-fluor--11β,17α-dihydroxy-16β-methyl-pregn-4-en-3,20--dion-17-propionat und 21-Chlor-6α-fluor-11β,17α--dihydroxy-16β-methyl-pregna-1,4-dien-3,20-dion--17-propionat.

Die neuen Verbindungen der Formel A besitzen wertvolle pharmakologische Eigenschaften, vor allem eine hervorragende antiinflammatorische Wirksamkeit, wie man sie bei z.B. lokaler Anwendung als Hemmung der entzündlichen Vorgänge feststellen kann. So erwies im Rattenohr-Dermatitis-Hemmtest nach Tonelli das 21-Chlor-6α-fluor-17α-hydroxy--16β-methyl-pregn-4-en-3,20-dion-17-propionat $ED_{50}$ (effektive Dosis, die eine 50%ige Hemmung der experimentellen Dermatitis verursacht) von 53 μg/ml, und das 21-Chlor-6α-fluor-11β,17α-dihydroxy-16β-methyl-pregn-4-en-3,20-dion-17-propionat $ED_{50}$ von 12 μg/ml. Dank dieser Eigenschaft sind die Verbindungen der Formel A in allen Indikationen, für die sich Glucocorticoid-Steroide mit entzündungshemmenden Eigenschaften eignen, insbesondere jedoch als topisch anzuwendende antiinflammatorische Glucocorticoide, z.B. zur Behandlung von entzündlichen Dermatosen, wie Ekzemen und Dermatiden, oder partiell corticoidresistenten Dermatosen, z.B. Psoriasis, verwendbar.

Zudem sind die Verbindungen der Formel A besonders wertvolle Zwischenprodukte zur Herstellung anderer nützlicher Stoffe, insbesondere anderer pharmakologisch wirksamer Steroide. In dieser Beziehung haben sie z.B. eine Schlüsselstellung bei der Synthese von antiinflammatorisch hochwirksamen, topisch anwendbaren Corticosteroiden, wie vom 21--Chlor-6α-fluor-9α-halogen-11β-hydroxy-16β-methyl-17α-propionyloxy-pregna-1,4-dien-3,20-dion und Analogen davon gemäss der Britischen Patentschrift 1 563 638. Zur Überführung in diese wertvollen Therapeutica benötigt man bei einer Verbindung der Formel A gegebenenfalls nur die problemlose konventionelle Einführung vom 9-Chlor oder 9-Fluor.

Die erwähnten neuen Verbindungen werden erfindungsgemäss hergestellt, indem man nacheinander a) in das 3β-Hydroxy-16-methyl-pregna-5,16-dien--20-on oder einen Carbonsäureester, wie insbesondere einen 3-Niederalkanoylester, davon Chlor in die 21-Stellung einführt, b) das erhaltene 21-Chlor-3β--hydroxy-16-methyl-pregna-5,16-dien-20-on zu einem 3-Niederalkanoylester verestert und mit einer Persäure zugleich in der 5,6- und 16,17-Stellung epoxidiert, c) in einem erhaltenen 5,6;16,17-Diepoxid den 16α,17α-Epoxidring unter Katalyse mit einer starken Säure zur 16-Methylen-17α-hydroxy--Gruppierung umlagert, den 5α,6α-Epoxidring mit Fluorwasserstoff in die 6β-Fluor-5α-hydroxy-Gruppierung überführt und die 5- und 17-Hydroxylgruppe verestert, d) den erhaltenen 21-Chlor-6β-fluor--3β,5α,17α-trihydroxy-16-methylen-pregnan-20--on-3-niederalkanoyl-5,17-diniederalkancarbonylester katalytisch hydriert, e) in der erhaltenen entsprechenden 16β-Methyl-Verbindung die veresterte 3-Hydroxylgruppe durch selektive Hydrolyse freisetzt und durch Behandlung mit einem Oxidationsmittel in die 3-Oxogruppe umwandelt, f) in einem erhaltenen 21-Chlor-6β-fluor-5α,17α-diniederalkancarbonyloxy-16β-methyl-pregnan-3,20-dion die 5-ständige Niederalkancarbonyloxygruppe sauer eliminiert und das 6β-Fluor unter Katalyse mit einer starken Säure in das 6α-Fluor-isomere umwandelt und, wenn ein Produkt der Formel A, worin Y für Hydroxyl steht, erwünscht ist, ein erhaltenes Endprodukt, worin Y für Wasserstoff steht, mittels des enzymatischen Systems eines 11β-hydroxylierenden Mikroorganismus in der 11β-Stellung hydroxyliert und/oder, wenn eine 1,2-Dehydroverbindung der Formel A erwünscht ist, ein erhaltenes 1,2-gesättigtes Endprodukt der Formel A dehydriert. Obwohl dabei alle einzelnen Verfahrensoperationen in an sich bekannter konventioneller Weise durchgeführt werden, treten bei einigen Stufen zusätzlich weitere, unerwartet günstige Resultate auf, die offensichtlich im Zusammenhang mit der besonderen Struktur der neuen Verbindungen stehen.

Für die erste Synthesestufe (bezeichnet als Stufe A in Schema I) des erfindungsgemässen Verfahrens, s.h. für die Einführung des 21ständigen Chloratoms, stehen einige allgemeine Verfahren zur Verfügung, wobei man zu diesem Zwecke einen reaktionsfähigen Ester der entsprechenden 21-Hydroxyverbindung, insbesondere das 21-Jodid oder -Sulfonat, wie 21-Mesylat, mit Lithium- oder Silberchlorid in einem polaren Lösungsmittel umsetzt. Vornehmlich erfolgt jedoch die Einführung des Chloratoms gemäss dem allgemeinen Verfahren des U.S. Pat. 3 758 524, indem man den bekannten Ausgangsstoff der Formel I unter der katalytischen Einwirkung eines Alkalimetallniederalkoholats, z.B. Natriumäthylats, Natriummethylats oder Kalium-tert-butylats, mit einem Niederalkyloxalat oder -formiat, z.B. Dimethyloxalat, Diäthyloxalat oder Äthylformiat, zum entsprechenden 21-Niederalkoxalylderivat, z.B. Methoxalyl- oder Äthoxalyl-derivat, (Formel II, X = -CO.CO.O.Niederalkyl) bzw. 21-Formylderivat (Formel II, X = -CH=O) umwandelt, aus diesem durch die Behandlung mit einem organischen Sulfonylazid, z.B. p-Tosylazid, das neue 21-Diazo-3β--hydroxy-16-methyl-pregna-5,16-dien-20-on bildet und dieses mit Chlorwasserstoff, unter Entstehung der entsprechenden 21-Halogenverbindung der Formel II (X=Cl) behandelt.

Diese wird anschliessend zum entsprechenden 3β-Niederalkanoylester der Formel III in an sich bekannter Weise verestert. Falls das entsprechende 3-Formiat [Formel III, Ac=HC(=O)-] erwünscht wird, kann man vornehmlich zur Veresterung überschüssige Ameisensäure, vorzugsweise eine etwa 85prozentige wässrige Ameisensäure, die zugleich auch als Lösungsmittel dient, verwenden und bei erhöhter Temperatur bis etwa 100°C arbeiten. Bei anderen Niederalkanoylestern, z.B. beim Acetat, behandelt man die 3-Hydroxyverbindung der Formel II in der üblichen Weise mit einem reaktionsfähigen Derivat der entsprechenden Niederalkansäure, wie einem Chlorid oder einem anderen gemischten Anhydrid, z.B. einem solchen mit Trifluoressigsäure, oder insbesondere dem symmetrischen Anhydrid, z.B. mit Acetanhydrid, in Gegenwart von mindestens einem Moläquivalent einer tertiären organischen Base und gegebenenfalls in einem aprotischen organischen Lösungsmittel im Temperaturbereich von etwa —10° bis etwa 30°, üblicherweise bei Zimmertemperatur. Vorzugsweise verwendet man dabei eine solche organische Base, die unfähig ist, z.B. infolge einer sterischen Hinderung, mit dem 21-Chloratom ein quaternäres Salz zu bilden; als eine solche Base wird bevorzugt z.B. ein 2,6-disubstituiertes, insbesondere dialkyliertes, Pyridinderivat, z.B. 2,4,6-Collidin oder vor allem 2,6-Lutidin, verwendet.

Die nächste Stufe (bezeichnet als B im Schema I.) des erfindungsgemässen Verfahrens besteht in der Epoxidierung des 5,16-Diens der Formel III mit einer organischen Persäure (Peroxysäure) oder einem analogen bekannten Epoxidierungsmittel. Die Reaktion wird in der konventionellen Weise bei Temperaturen von etwa —15° bis etwa +30°, insbesondere zwischen etwa 0° und Zimmertemperatur in einem inerten organischen Lösungsmittel, insbesondere einem Äther, wie Diäthyläther, 1,2-Dimethoxyäthan oder Tetrahydrofuran, oder einem halogenierten Kohlenwasserstoff, wie Chloroform oder Methylenchlorid, vorzugsweise in Abwesenheit von Wasser, durchgeführt. Als Epoxidierungsmittel verwendet man vornehmlich eine gegebenenfalls substituierte Peroxybenzoesäure, wie Perbenzoesäure, Phthalmonoperoxysäure («Perphthalsäure») oder insbesondere die m-Chlorperbenzoesäure. Das Reaktionsmittel greift gleichzeitig an 2 Stellen des Moleküls ein: während die Epoxidierung der 16,17-Doppelbindung mit einer hohen Stereospezifität verläuft und praktisch ausschliesslich zum 16α,17α-Epoxidring führt, attakiert das Epoxidierungsmittel die 5,6-Doppelbindung von beiden Seiten; demzufolge resultiert neben dem als Hauptprodukt auftretenden 5α,6;16α,17-Diepoxid der Formel IVa als Nebenporodukt im Verhältnis von etwa 4 : 1 das epimere 5β,6;16α17-Diepoxid der Formel IVb. Die Epimeren lassen sich durch die üblichen physikalischen Methoden, wie Kristallisation und/oder Chromatographie, leicht trennen und werden auch getrennt in der nächsten Stufe verarbeitet.

Die weitere Stufe (bezeichnet als C in Schema I.) des erfindungsgemässen Verfahrens umfasst die säurekatalysierte Umlagerung des 16α,17-Epoxidringes in die 16-Methylen-17α-hydroxy-Gruppierung, sowie die Umwandlung des 5α,6-Epoxidringes in die 6β-Fluor-5α-hydroxy-Gruppierung. Besonders vorteilhaft werden beide Umwandlungen in einer einzigen Operation durchgeführt, indem man ein entsprechendes 5α,6;16α,17-Diepoxid der Formel IVa mit Fluorwasserstoff behandelt, welcher nicht nur das notwendige Fluoridanion für die 6β-Fluorierung, sondern auch die für die Umlagerung des 16,17-Epoxids notwendige Acidität gleichzeitig besitzt. Die Reaktion kann mit etwa 40prozentigem wässrigem, oder vorzugsweise mit wasserfreiem flüssigem Fluorwasserstoff z.B. unter solchen Bedingungen durchgeführt werden, die bei der konventionellen Überführung eines 9β,11-Epoxids in die entsprechende 9α-Fluor-11β-hydroxy-Verbindung gebräuchlich sind. Man arbeitet zweckmässig in überschüssigem HF als Lösungsmittel, gegebenenfalls auch in Gegenwart eines inerten Lösungsmittels, wie Chloroform, Tetrahydrofuran, Dioxan, oder insbesondere Dimethylformamid. Fluorwasserstoff kann man auch in Form eines fluorwasserstoffabgebenden Mittels einsetzen, z.B. als ein Salz mit einer tertiären organischen Base, oder insbesondere als eine ähnliche Additionsverbindung, z.B. ein Addukt mit einem Carbaminsäure- oder Thiocarbaminsäure-Derivat, insbesondere als Addukt mit Harnstoff gemäss U.S. Patent 3 211 758. Überraschenderweise ergibt diese Reaktion als das einzige Isomere die 16-Methylen-17α-hydroxy-Verbindung der Formel V, die Bildung des $\Delta^{15}$-16-Methyl-17α-hydroxy-Isomeren, welches bei der Umlagerung eines 16β-Methyl-16α,17α-epoxids mit Fluorwasserstoff oft als das Hauptprodukt entsteht, konnte im vorliegenden Fall nicht festgestellt werden.

Zur erfindungsgemässen Umwandlung eines epimeren 5β,6;16α,17-Diepoxids der Formel IVb ins Fluorhydrin der Formel V werden mehrere Zwischenstufen gebraucht, da man zunächst die ungünstige β-Konfiguration des 5,6-Epoxid-Ringes in die gewünschte α-Konfiguration umwandeln muss. Die mehrstufige Umwandlung wird gemäss Schema II durchgeführt, indem man das entsprechende 5β,6;-16α,17-Diepoxid der Formel IVb mit einer starken sauerstoffhaltigen Säure umsetzt, die erhaltene 5α,6β,17α-Trihydroxy-16-methylen-Verbindung der Formel XI mit Methansulfonylchlorid oder einem ähnlichen organischen Sulfonylhalogenid in Gegenwart einer organischen Base behandelt und die erhaltene 5α,6-Epoxy-17α-hydroxy-16-methylen-Verbindung XII, die die richtige α-Konfiguration des Epoxid-Rings besitzt, mit Fluorwasserstoff umsetzt und so zum Fluorhydrin der Formel V überführt. Die Umsetzung mit einer starken Säure (Stufe $C_a'$ im Schema II) erfolgt in der herkömmlichen Weise z.B. in Anwesenheit von kleinen Mengen Wasser in einem organischen Lösungsmittel, wie einem Niederalkanol, z.B. Methanol oder Äthanol, Äther, z.B. Diäthyläther oder insbesondere Tetrahydrofuran oder Dioxan, oder einem halogenierten Kohlenwasserstoff, z.B. Chloroform, oder in einem Gemisch davon; als Säure verwendet man eine anorganische Säure, wie Perchlorsäure oder insbesondere Schwefelsäure, oder eine organische Sulfonsäure, wie insbesondere die p-Toluolsulfonsäure. Die Umsetzung kann man mit katalytischen Mengen der Säure im breiten Temperaturenbereich bis zur Siedetemperatur des Reaktionsgemisches durchführen, vorzugsweise wird aber schonend bei Zimmertemperatur gearbeitet. Für die zweite Stufe ($C_b'$ im Schema II), d.h. die Behandlung mit einem Sulfonylhalogenid, werden analoge Bedingungen angewendet, wie oben für die basenkatalysierte Veresterung der 3-Hydroxylgruppe geschildert wurde, mit einer besonderen Vorsicht bei der Auswahl einer geeigneten Base. Die dritte Stufe (als $C_c'$ im Schema II bezeichnet), d.h. die Aufspaltung des 5α,6α-Epoxid-Rings in der Verbindung der Formel XII unter Bildung des entsprechenden Fluorhydrins der Formel V, wird in derselben Weise durchgeführt, wie oben für die Behandlung des Diepoxids IVa mit Fluorwasserstoff eingehend angegeben wird. Der ganze dreistufige Prozess ist von Vorteil in erster Linie für die Durchführung des erfindungsgemässen Verfahrens im technischen Massstab als eine Eventualmassnahme zur Verwertung des Nebenproduktes der Formel IVb und zur zusätzlichen Erhöhung der Gesamtausbeute des Zwischenproduktes der Formel V.

Anschliessend an die Aufspaltung der Diepoxide IVa bzw. IVb werden im erhaltenen 21-Chlor-6β-fluor-5α,17α-dihydroxy-3β-niederalkanoyloxy-16-methylen-pregnan-20-on die beiden freien Hydroxylgruppen mit dem oben definierten Rest-COR einer Niederalkancarbonsäure unter Bildung des entsprechenden 3,5,17-Triesters der Formel VI verestert. Dazu verwendet man an sich bekannte, herkömmliche Methoden zur Veresterung von schwierig veresterbaren tertiären Hydroxylgruppen, z.B. die Behandlung der Verbindung der Formel V, die je eine solche Hydroxylgruppe in der 5α- und 17α-Stellung hat, mit einem symmetrischen Anhydrid einer geeigneten Niederalkalicarbonsäure, z.B. mit Propionsäureanhydrid, unter Katalyse mittels einer starken Mineralsäure, wie insbesondere der Perchlorsäure, oder einer organischen Sulfonsäure, wie p-Toluolsulfonsäure. Vornehmlich verwendet man jedoch als Veresterungsmittel ein reaktionsfähiges gemischtes Anhydrid der entsprechenden Niederalkancarbonsäure, insbesondere ein solches mit Trifluoressigsäure, z.B. das gemischte Propionsäure-Trifluoressigsäure-Anhydrid. Die Umsetzung erfolgt üblicherweise bei Zimmertemperatur unter Ausschluss von Wasser in einem inerten organischen Lösungsmittel, wie einem gegebenenfalls halogenierten Kohlenwasserstoff, z.B. Benzol, Toluol, Cyclohexan, bzw. Chloroform oder Methylenchlorid, oder einem Äther, wie Diäthyläther, Dioxan oder Tetrahydrofuran, mit überschüssigem Veresterungsmittel. Dieses wird vorteilhaft unmittelbar vor der Umsetzung im Reaktionsgemisch vorbereitet, indem man die entsprechende Niederalkancarbonsäure mit einer etwa äquivalenten Menge Trifluoracetanhydrid, gegebenenfalls unter Kühlung, vermischt und 30-60 Minuten bei Zimmertemperatur reagieren lässt.

Die nächste Stufe (bezeichnet als Stufe D im Schema I) des erfindungsgemässen Verfahrens besteht in der katalytischen Hydrierung eines 16-Methylenderivates der Formel VI zur entsprechenden 16β-Methyl-Verbindung der Formel VII. Die Hydrierung wird in der herkömmlichen Weise, z.B. mit elementarem Wasserstoff bei atmosphärischem oder leicht erhöhtem (bis zu etwa 5 Atm) Druck bei Temperaturen in der Umsetzung der Zimmertemperatur in den üblichen organischen Lösungsmitteln, wie Äthern, z.B. 1,2-Dimethoxyäthan, Dioxan oder Tetrahydrofuran, Niederalkanolen, z.B. Methanol oder Äthanol, oder niederaliphatischen Estern, z.B. Äthylacetat, durchgeführt. Als Katalysator kann man die üblichen fein verteilten Metallkatalysatoren verwenden, wie die Raney-Metalle, insbesondere Raney-Nickel, oder Edelmetalle, z.B. Rhodium oder ganz besonders bevorzugt Platin, die auch auf einem geeigneten Trägermaterial, wie Kieselgel oder Aluminiumoxid, feinverteilt sein können. Als einzige besondere Massnahme der Hydrierung ist darauf zu achten, dass das 21ständige Halogenatom nicht wegreduziert wird, es sind insbesondere zu energische Bedingungen, wie erhöhte Temperatur und basisch reagierende Mittel, zu meiden. Es ist äusserst überraschend, dass diese Hydrierung praktisch ausschliesslich das 16β-Methyl-Epimere liefert, obwohl bei den bekannten analogen Fällen jeweils beträchtliche Mengen des 16α-Methyl-Epimeren gleichzeitig gebildet wurden.

Bei der weiteren Stufe (bezeichnet als Stufe E in Schema I) des erfindungsgemässen Verfahrens wird im 21-Chlor-6β-fluor-3β,5α,17α-trihydroxy-16β-methyl-pregnan-20-on 3-Niederalkanoyl-5,17-diniederalkancarbonylester der Formel VII zunächst die veresterte 3β-Hydroxylgruppe hydrolytisch freigesetzt (womit der 5,17-Diniederalkancarbonylester der Formel VIII entsteht) und anschliessend zur 3-Oxogruppe oxidiert. Die selektive hydrolytische Freisetzung der sekundären 3β-Hydroxylgruppe in Gegenwart von analog veresterten tertiären Hydroxylgruppen in den Stellungen 5α- und 17α- wird in an sich bekannter Weise unter Säurekatalyse durchgeführt, z.B. in einem Niederalkanol, wie Methanol, Äthanol oder Isopropylalkohol in Gegenwart einer Mineralsäure, wie Chlorwasserstoffsäure oder Schwefelsäure. Falls die 3-Hydroxylgruppe im Ausgangsstoff der Formel VII als Formiat vorliegt, ist die selektive Freisetzung besonders glatt und kann auch mit schwach basischen Mitteln, z.B. mit einem Äquivalent Alkalimetallhydrocarbonat, wie Natrium- oder Kaliumhydrogencarbonat bei Raumtemperatur erfolgen. Auch die anschliessende Oxydation (Dehydrierung) der freien 3β-Hydroxylgruppe zur Oxogruppe unter Bildung des 21-Chlor-6β-fluor-5α,17α-diniederalkancarbonyloxy-16β-methyl-pregnan-3,20-dions der Formel IX geschieht in der konventionellen, allgemein bekannten Weise, z.B. mit einer Verbindung des 6-wertigen Chroms, wie Chromtrioxid oder Chromsäure und ihre Alkalimetallsalze, wobei man als Reaktionsmedium Niederalkancarbonsäuren, wie Essig- oder Propionsäure, oder ein Keton, wie Aceton, gegebenenfalls unter Verdünnung mit einem halogenierten Niederalkan, wie Dichlormethan oder Chloroform, verwendet und die Reaktionstemperatur vorzugsweise unterhalb der Raumtemperatur hält. Eine bevorzugte Variante ist die Oxydation mit einer Lösung von Chromtrioxid in wässriger Schwefelsäure (Jones-Reagens), die man üblicherweise in Aceton bei einer Temperatur zwischen etwa —10° bis etwa 25°, vorzugsweise in der Umgebung des Nullpunktes durchführt.

Die nächste Stufe (bezeichnet als Stufe F im Schema I) des erfindungsgemässen Verfahrens besteht in der sauren β-Eliminierung der veresterten 5α-Hydroxylgruppe des 3-Ketons der Formel IX unter Bildung der mit der Oxogruppe konjugierten 4,5-Doppelbindung und in der säurekatalysierten Isomerisierung des 6β-ständigen Fluoratoms in die thermodynamisch stabilere 6α-Konfiguration, wodurch das 21-Chlor-6α-fluor-17α-niederalkancarbonyloxy-16β-methyl-pregn-4-en-3,20-dion der Formel X resultiert. Zur β-Eliminierung der 5-ständigen Niederalkancarbonyloxygruppe genügt schon die geringe Acidität einer Carbonsäure; in an sich bekannter Weise sind zu diesem Zweck flüssige Carbonsäuren, die zugleich auch als Lösungsmittel dienen, besonders geeignet, darunter insbesondere niederaliphatische Monocarbonsäuren, wie vor allem Eisessig. Vorzugsweise wird bei einer erhöhten Temperatur von etwa 50° bis zur Siedetemperatur des Reaktionsgemisches gearbeitet. Zu einer vollständigen Isomerisierung des 6ständigen Fluoratoms reicht jedoch die Acidität der erwähnten Säuren meistens nicht aus; dann ist es nötig, das Produkt einer derartigen Eliminierung noch einer nachträglichen Isomerisierung durch eine stärkere Säure zu unterziehen. Indem starke Säuren die β-Eliminierung bei vorsichtiger Anwendung nicht beeinträchtigen, kann man vorteilhaft beide Umwandlungen zu einer einzigen Operation zusammenschliessen, indem man in an sich bekannter Weise die Verbindung der Formel IX mit einer katalytischen Menge einer starken Säure in einem inerten organischen Lösungsmittel behandelt. Als Säure verwendet man entweder anorganische Säuren, z.B. Schwefelsäure, Perchlorsäure oder eine Halogenwasserstoffsäure, wie insbesondere Chlorwasserstoff oder Bromwasserstoff, oder starke organische Säuren, z.B. Sulfonsäuren, wie insbesondere p-Toluolsulfonsäure. Als Lösungsmittel kommen z.B. die oben erwähnten flüssigen Carbonsäuren, insbesondere Eisessig, und halogenierte Kohlenwasserstoffe, wie Chloroform und Methylenchlorid, sowie ihre Gemische, besonders in Betracht. Die Reaktionstemperatur liegt üblicherweise etwa zwischen dem Nullpunkt und der Zimmertemperatur. Vornehmlich wird in einer Chloroformlösung mit gasförmigem trockenem Chlorwasserstoff bei etwa 0° umgesetzt.

Die erhaltenen 1,2-gesättigten 11-unsubstituierten Endstoffe des oben beschriebenen erfindungsgemässen Verfahrens, welche durch die Formel A charakterisiert sind, worin Y Wasserstoff bedeutet und worin sich eine einfache Bindung in 1,2-Stellung befindet, kann man anschliessend, wenn erwünscht, durch zusätzliche Strukturmerkmale modifizieren, indem man die 11β-Hydroxylgruppe und/oder die 1,2-Doppelbindung in beliebiger Reihenfolge einführt.

Zwecks der gewünschtenfalls durchzuführenden Einführung der 11β-Hydroxylgruppe wird gemäss Stufe G im Schema I des erfindungsgemässen Verfahrens das 21-Chlor-6α-fluor-17α-niederalkancarbonyloxy-16β-methyl-pregn-4-en-3,20-dion oder -1,4-dien-3,20-dion der Formel Aa einer biologischen 11β-Hydroxylierung zum 21-Chlor-6α-fluor-11β-hydroxy-17α-niederalkancarbonyloxy-16β-methyl-pregn-4-en-3,20-dion bzw. -1,4-dien-3,20-dion (21-Chlor-6α-fluor-11β,17α-dihydroxy-16β-methyl-pregn-4-en-3,20-dion 17-Niederalkancarbonylester bzw. dessen 1,2-Dehydroanaloge) der Formel Ab unterworfen. Die Hydroxylierung wird nach den an sich bekannten Methoden der biologischen Transformation durchgeführt, die zur Einführung der 11β-Hydroxygruppe bei Steroiden der Pregnan-Reihe allgemein gebraucht werden, und erfolgt mittels des entsprechenden Enzymsystems eines bekannten 11β-hydroxylierenden Mikroorganismus, wie Aspergillus niger, Cunninghamella blakesleana oder insbesondere Curvularia lunata. Diese 11β-Hydroxylierung ist im vorliegenden Fall mit einem unerwarteten wesentlichen Vorteil verbunden: die veresterte 17α-Hydroxylgruppe wird während der Transformation nicht freigesetzt, wie es bei solchen mikrobiologischen Prozessen allgemein üblich ist, sondern bleibt unversehrt erhalten. Das hydroxylierende Enzymsystem kann sich bei dieser Transformation direkt in der Zelle eines lebenden Mikroorganismus befinden und wirkt bei einer Variante des Hydroxylierungsverfahrens während der Kultivierung des Mikroorganismus in einem zweckmässigen, herkömmlichen Nährmedium oder bei einer anderen Variante in einer ruhenden Kultur, die man z.B. durch mechanisches Abtrennen des angewachsenen Mikroorganismus von der Nährlösung und Suspendieren in einem nährstofffreien wässrigen Medium zubereitet. Bekanntlich kann man aber auch mit einem hydroxylierenden Enzymsystem in einer zellfreien Form einwirken, z.B. mit einem Produkt, das durch eine schonende Tötung und/oder Zerstörung der Zellenwand des Mikroorganismus resultiert, oder mit einem mehr oder weniger angereicherten bzw. gereinigten Enzympräparat. Die Isolierung des hydroxylierten Steroids der Formel A aus dem Substrat erfolgt in der herkömmlichen Weise, gewöhnlich durch Extraktion mit geeigneten organischen Lösungsmitteln, z.B. halogenierten Kohlenwasserstoffen oder niederaliphatischen Estern, wie Chloroform oder Methylenchlorid, bzw. Äthylacetat.

Die gewünschtenfalls durchzuführende nachträgliche Einführung der 1,2-Doppelbindung in die 1,2-gesättigten Verbindungen unter Entstehung von entsprechenden 1,2-Dehydroderivaten erfolgt in an sich bekannter Weise, z.B. durch Dehydrieren. Man kann dazu biologische Dehydrierungsverfahren anwenden, z.B. mittels der Mikroorganismen Corynebacterium simplex oder Septomyxa affinis oder ihrer Enzymsysteme dehydrieren, oder mit Selendioxid in einem organischen Lösungsmittel, z.B. tert-Butylalkohol, behandeln. Vorzugsweise lässt man jedoch mit 2,3-Dichlor-5,6-dicyan-1,4-benzochinon etwa bei Siedehitze während mehreren, z.B. 6-24 Stunden einwirken; als Lösungsmittel verwendet man übliche Lösungsmittel, z.B. aromatische Kohlenwasserstoffe, wie Benzol oder Xylol, niederaliphatische Alkohole, wie Äthanol, Propanol oder tert-Butylalkohol, niederaliphatische Ketone, wie Acetone oder 2-Butanon, aliphatische Ester, wie Äthylacetat, oder cyclische Äther, wie Dioxan oder Tetrahydrofuran.

Im oben geschilderten erfindungsgemässen Verfahren werden vorzugsweise Reaktionsmittel und Zwischenprodukte verwendet, die zu den besonders hervorgehobenen, insbesondere den spezifisch genannten, Endstoffen und Zwischenprodukten führen.

Sofern nicht spezifisch definiert wird, bezieht sich in der ganzen Beschreibung die Bezeichnung «nieder» im Zusammenhang mit einem Kohlenwasserstoffrest auf einen solchen mit höchstens 7 Kohlenstoffatomen.

Die Erfindung betrifft auch diejenigen Ausführungsformen der obigen Verfahren, bei denen man von einer auf irgendeiner Stufe als Zwischenprodukt erhältlichen Verbindung ausgeht und die fehlenden Schritte durchführt, oder bei denen ein Ausgangsstoff unter den Reaktionsbedingungen gebildet wird.

Die vorliegende Erfindung betrifft auch pharmazeutische Präparate für Menschen und Säugetiere,

welche die neuen oben beschriebenen Verbindungen der Formel A in einer therapeutisch wirksamen Menge als aktive Substanzen zusammen mit einem pharmazeutischen Trägermaterial enthalten, sowie ihre Herstellung. Als Träger verwendet man organische oder anorganische Stoffe, die für die enterale, insbesondere orale, und intrauterine, parenterale, oder topicale Gabe geeignet sind. Für die Bildung derselben kommen solche Stoffe in Frage, die mit den neuen Verbindungen nicht reagieren, wie z.B. Wasser, Gelatine, Milchzucker, Stärke, Magnesiumstearat, Talk, pflanzliche Öle, Benzylakohol, Gummi, Polyalkylenglykole, Vaseline, Cholesterin und andere bekannte Arzmeimittelträger. Die pharmazeutischen Präparate können in fester Form, z.B. als Tabletten, Dragées oder Kapseln, oder in flüssiger oder halbflüssiger Form als Lösungen, Suspensionen, Emulsionen, Salben oder Cremen vorliegen. Gegebenenfalls sind diese pharmazeutischen Präparate sterilisiert und/oder enthalten Hilfsstoffe, wie Konservierungs-, Stabilisierungs-, Netz- oder Emulgiermittel, Salze zur Veränderung des osmotischen Druckes oder Puffer. Sie können auch noch andere therapeutisch wertvolle oder biologisch wirksame Stoffe enthalten.

Dabei kommen in erster Linie topisch anwendbare pharmazeutische Präparate, wie Crèmen, Salben, Pasten, Schäume, Tinkturen und Lösungen, in Frage, die von etwa 0,01% bis etwa 0,5% des Wirkstoffs enthalten.

Crèmen sind Öl-in-Wasser-Emulsionen, die mehr als 50% Wasser aufweisen. Als ölige Grundlage verwendet man in erster Linie Fettalkohole, z.B. Lauryl-, Cetyl- oder Stearylalkohole, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Äthylenoxidaddukte davon, wie Polyglycerin-fettsäureester oder Polyoxyäthylensorbitan-fettsäureester (Tweens), ferner Polyoxyäthylen-fettalkoholäther oder -fettsäureester, oder entsprechende ionische Emulgatoren, wie Alkalimetallsalze von Fettalkoholsulfaten, z.B. Natriumlaurylsulfat, Natriumacetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Crème vermindern, z.B. Polyalkohole, wie Glycerin, Sorbit, Propylenglykol und/oder Polyäthylenglykole, ferner Konservierungsmittel und Riechstoffe.

Salben sind Wasser-in-Öl-Emulsionen, die bis zu 70%, vorzugsweise jedoch von etwa 20% bis etwa 50% Wasser oder wässrige Phase enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole, bzw. Wollwachs, enthalten. Emulgatoren sind entsprechende lipophile Substanzen, wie Sorbitan-fettsäureester (spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyäthylenglykol, sowie Konservierungsmittel und Riechstoffe.

Fettsalben sind wasserfrei und enthalten als Grundlage insbesondere Kohlenwasserstoffe, z.B. Paraffin, Vaseline und/oder flüssige Paraffine, ferner natürliche oder partial-synthetische Fette, z.B. Kokosfettsäuretriglycerid, oder vorzugsweise gehärtete Öle, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmefähigkeit steigernden Fettalkohole, Emulgatoren und/oder Zusätze.

Pasten sind Crèmen und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorliegende flüssige Öl-in-Wasser-Emulsionen, wobei halogenierte Kohlenwasserstoffe, wie Chlorfluorniederalkane, z.B. Dichlordifluormethan und Dichlortetrafluoräthan, als Treibmittel verwendet werden. Als Ölphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyäthylen-sorbitan-fettsäureester (Tweens), und solchen mit vorwiegend lipophilen Eigenschaften, wie Sorbitanfettsäureester (Spans). Dazu kommen die üblichen Zusätze, wie Konservierungsmittel.

Tinkturen und Lösung weisen meistens eine wässerig-äthanolische Grundlage auf, der u.a. Polyalkohole, z.B. Glycerin, Glykole, und/oder Polyäthylenglykol, als Feuchthaltemittel zur Herabsetzung der Verdunstung, und rückfettende Substanzen, wie Fettsäureester mit niedrigen Polyäthylenglykolen, d.h. im wässrigen Gemisch lösliche, lipophile Substanzen als Ersatz für die der Haut mit dem Äthanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel vor der Emulgierung in einer der beiden Phasen gelöst; bei Verarbeitung als Suspension wird er nach der Emulgierung mit einem Teil der Grundlage vermischt und dann dem Rest der Formulierung beigegeben.

Die Dosierung des Wirkstoffs, z.B. der oben besonders hervorgehobenen Verbindungen, erfolgt im Prinzip analog derjenigen von anerkannten topischen Antiinflammatorika vom Corticoid-Typ; sie hängt jedoch auch einerseits von Spezies, Körpergewicht, Alter und individuellem Zustand des Warmblüters, andererseits von der Applikationsweise ab, und kann

im Routine-Test in bekannter Weise für jeden individuellen Fall festgestellt werden.

Die Erfindung betrifft auch eine Methode zur Linderung oder Behebung von krankhaften entzündlichen Zuständen des Körpers, und insbesondere der Haut, eines Warmblüters, vor allem des Menschen, welche durch die Behandlung dieses Körpers oder Körperteils, vorzugsweise in topischer Applikation, mit einer antiinflammatorisch wirksamen Menge einer Verbindung der Formel A, allein oder in Form eines pharmazeutischen Präparats, charakterisiert ist. Unter der Bezeichnung «eine antiinflammatorisch wirksame Menge» ist eine solche Menge des Wirkstoffes zu verstehen, die zu einer signifikanten Inhibition der Entzündung ausreicht.

In den nachfolgenden Beispielen wird die praktische Durchführung der vorliegenden Erfindung noch näher illustriert, ohne sie dadurch in ihrem Umfang einzuschränken. Die Temperaturen werden vor- und nachstehend in Celsiusgraden angegeben.

*Schema I.*

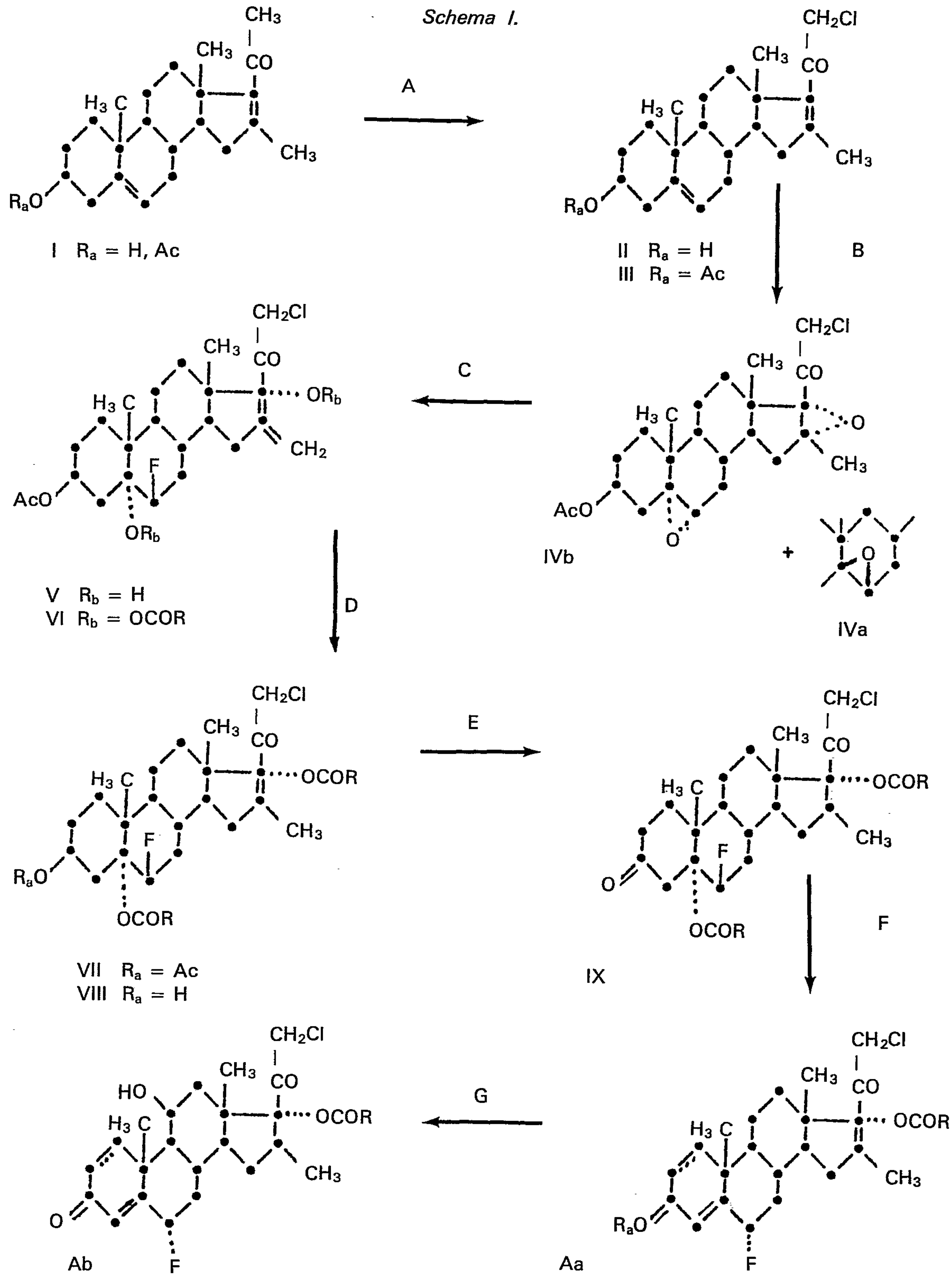


I $R_a$ = H, Ac

II $R_a$ = H

III $R_a$ = Ac

IVb + IVa

V $R_b$ = H

VI $R_b$ = OCOR

VII $R_a$ = Ac

VIII $R_a$ = H

IX

Ab

Aa

*Schema II.*

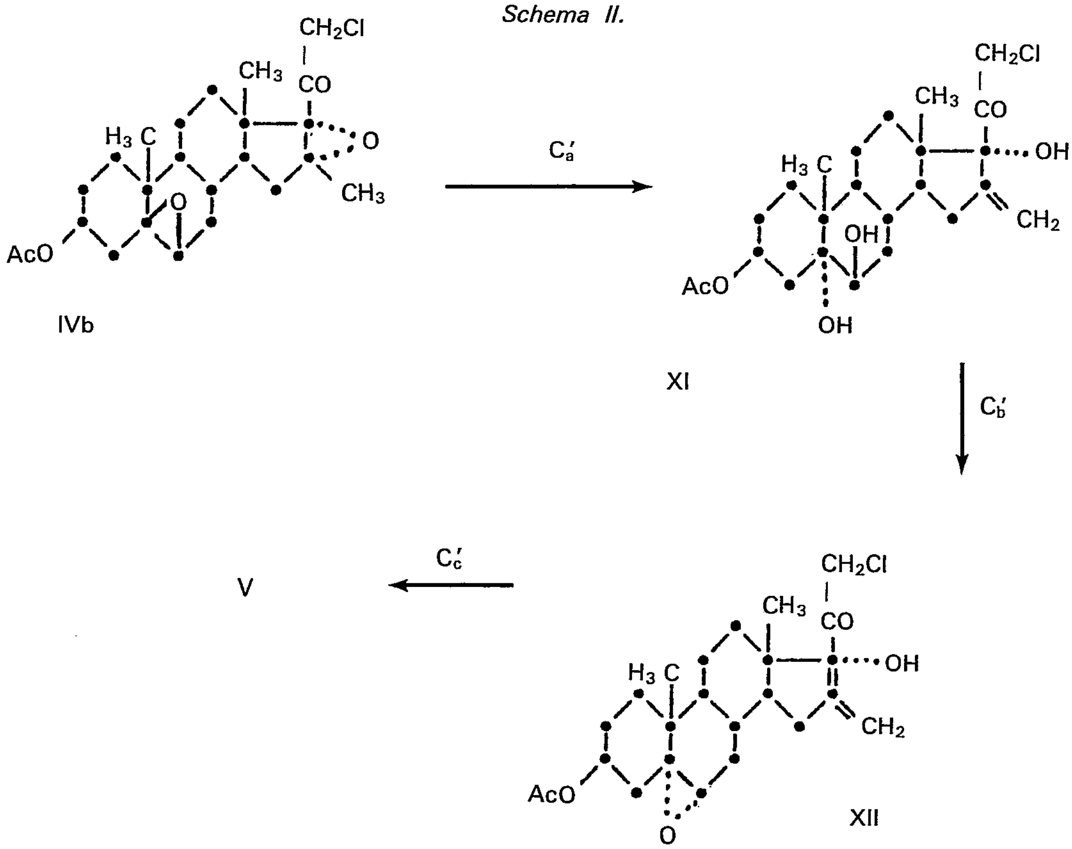


IVb

XI

XII

V

*Beispiel 1*

Zu 943 ml einer gerührten Lösung, zubereitet durch Verdünnen von 200 g Oxalsäuredimethylester mit Toluol bis zum Gesamtvolumen von 1 Liter, gibt man unter Stickstoffstrom und Eiskühlen 94,3 g pulverförmiges Natriummethylat, spült mit 900 ml Toluol nach, versetzt mit 117,6 g kristallinem 3β-Hydroxy-16-methyl-pregna-5,16-dien-3-on-acetat, spült mit weiteren 900 ml Toluol nach und lässt sieben Stunden bei Zimmertemperatur rühren. Das Gemisch wird unter Eiskühlung nacheinander mit 64 ml Eisessig, 136,5 ml Triäthylamin und 194,2 g p-Toluolsulfonylazid unter Nachspülen mit 585 ml Toluol versetzt, die Kühlung entfernt und das Gemisch 18 Stunden bei Zimmertemperatur stehen gelassen. Das Reaktionsgemisch wird auf halbgesättigte Kochsalzlösung geleert, die organische Schicht abgetrennt und die wässrige nochmals mit Chloroform extrahiert; kombinierte organische Lösungen werden dann mit halbgesättigter Kochsalzlösung gewaschen, getrocknet und im Vakuum eingedampft. Der gelbe kristalline Rückstand wird während drei Stunden mit 5,8 ℓ tert-Butylalkohol und 1,17 ℓ 2N-Kaliumhydroxid-Lösung verrührt, auf eine halbgesättigte Kochsalzlösung geleert und wie vorangehend aufgearbeitet. Der Rückstand wird in Methylenchlorid gelöst und durch 560 g Aluminiumoxid (Aktivität II) filtriert. Die mit 10 ℓ Methylenchlorid und 30 ℓ eines Gemisches (V/V) von Toluol-Äthylacetat (4 : 1) ausgewaschenen Eluate werden im Vakuum eingedampft. Durch Umlösen des Rückstandes aus Methylenchlorid-Äther erhält man 103 g 21-Diazo-3β-hydroxy-16-methyl-pregna-5,16-dien-20-on vom Smp. 137-139° (Zers.).

*Beispiel 2*

Zu einer Mischung von 103 g 21-Diazo-3β-hydroxy-16-methyl-pregna-5,16-dien-20-on, 1,73 ℓ Methylenchlorid und 1,73 ℓ Äther gibt man unter Rühren bei —10° im Stickstoffstrom 3,42 ℓ einer 3N-Lösung von Chlorwasserstoff in Äther, wobei die Temperatur auf etwa 2° steigen kann. Nach 15 Minuten wird das Kühlbad entfernt, das Gemisch auf eine Lösung 1 kg Natriumacetat in 5 ℓ Wasser geleert, in Methylenchlorid aufgenommen, mit gesättigter Natriumhydrogencarbonatlösung gewaschen, getrocknet und im Vakuum eingedampft. Durch Kristallisation des Rückstandes aus Methanol erhält man 87,65 g 21-Chlor-3β-hydroxy-16-methyl-pregna-5,16-dien-20-on vom Smp. 170-172°.

*Beispiel 3*

Eine Mischung von 86 g 21-Chlor-3β-hydroxy-16-methyl-pregna-5,16-dien-20-on, 800 ml Acetanhydrid und 172 ml 2,6-Lutidin wird während 137 Stunden im Dunkeln bei Zimmertemperatur gerührt, auf 4 kg Eis und 7,2 ℓ Wasser gegossen und noch während zwei weiteren Stunden gerührt. Der Niederschlag wird abgenutscht, mit Wasser gewaschen, in Methylenchlorid gelöst, getrocknet und im Vakuum eingedampft. Den Rückstand filtriert man in Toluollösung durch 430 g Kieselgel und wäscht mit 18 ℓ Toluol aus. Der nach Eindampfen der Eluate im Vakuum erhaltene Rückstand (90 g) kann in der nächsten Stufe direkt verarbeitet werden. Nach Umlösen einer Probe aus Methylenchlorid-Äther-Pentan schmilzt das gereinigte 21-Chlor-3β-hydroxy-16-methyl-pregna-5,16-dien-20-on-acetat bei 183-186°.

*Beispiel 4*

Zu 91,45 g rohem 21-Chlor-3β-hydroxy-16-methyl-pregna-5,16-dien-20-on-acetat und 1,26 ℓ Methylenchlorid gibt man unter Rühren und Kühlen mit Eiswasser 126 g m-Chlorperbenzoesäure. Nach 18stündigem Rühren bei Zimmertemperatur wird das Gemisch auf 3 ℓ gesättigte Natriumhydrogencarbonatlösung gegossen, durch mehrmaliges Extrahieren in Methylenchlorid aufgenommen, nacheinander mit 2,5 ℓ 1N-Natriumthiosulfat-Lösung und gesättigter Natriumhydrogencarbonatlösung gewaschen, getrocknet und im Vakuum eingedampft. Der Rückstand wird an 920 g Kieselgel chromatographiert. Die ersten, mit insgesamt 7,5 ℓ Toluol eluierten, Fraktionen werden verworfen, die nachfolgenden im Vakuum eingedampft. Die erhaltenen Rückstände werden vereinigt und aus Methylenchlorid-Äther umkristallisiert; das resultierende 70,36 g 21-Chlor-5α,6;-16α,17-diepoxy-3β-hydroxy-16β-methylpregnan-20-on-acetat ist nur mit sehr wenig des entsprechenden 5β,6-Epoxids verunreinigt und wird in dieser Reinheit weiter verarbeitet. Eine Probe schmilzt nach chromatographischer Reinigung bei 212-214°.

Aus der Mutterlauge vom 5α,6-Epoxid erhält man nach Kristallisation aus Methylenchlorid-Äther 18,6 g 21-Chlor-5β,6;16α,17-diepoxy-3β-hydroxy-16β-methyl-pregnan-20-on-acetat, dem man nur geringe Mengen des entsprechenden 5α,6-Epoxids anhaften und das in dieser Reinheit weiter verarbeitet wird. Nach mehrmaligem Umlösen aus Methylenchlorid-Äther schmilzt eine Probe bei 158-160°.

*Beispiel 5*

Zu 13 g 21-Chlor-5β,6;16α,17-diepoxy-3β-hydroxy-16β-methyl-pregnan-20-on-acetat gibt man 1,3 ℓ einer Mischung von 1,8 ℓ Dioxan und 54 ml konzentrierter Schwefelsäure. Nach sechs Stunden wird das Gemisch auf gesättigte Natriumhydrogencarbonatlösung gegossen und mehrmals mit einer Mischung (V/V) von Chloroform-Alkohol (7 : 3) extrahiert. Die organischen Auszüge werden mit gesättigter Kochsalzlösung gewaschen, getrocknet und im Vakuum eingedampft. Man erhält 13,15 g rohes 21-Chlor-3β,5α,6β,17α-tetrahydroxy-16-methylen-pregnan-20-on-3-acetat in Form eines Schaumes. Dieses wird in 167 ml 2,6-Lutidin gelöst und unter Rühren und Eiskühlung mit 6,13 ml Methansulfonylchlorid versetzt und 144 Stunden bei 0-5° gerührt. Das Gemisch wird mit Eis versetzt und eine weitere Stunde verrührt, mit 300 ml Eisessig angesäuert, in mehrere Portionen von Methylenchlorid aufgenommen, mit gesättigter Natriumhydrogencarbonatlösung gewaschen, getrocknet und im Vakuum eingedampft. Den Rückstand chromatographiert man an 260 g Kieselgel, wobei man mit Gemischen (V/V) von Toluol-Äthylacetat (97 : 3) und (95 : 5) das 21-Chlor-5α,6-epoxy-3β,17α-dihydroxy-16-methylen-pregnan-20-on-acetat (5,2 g), Smp. 232-234° eluiert.

*Beispiel 6*

Ein gemisch von 7,5 g 21-Chlor-5α,6-epoxy-3β,-17α-dihydroxy-16-methylen-pregnan-20-on-3-acetat in 150 ml einer vorher zubereiteten Mischung von 56 Gewichtsteilen Fluorwasserstoff und 44 Gewichtsteilen Hanrstoff wird während 90 Minuten bei Zimmertemperatur gerührt, auf 2,25 kg Eis und 1,13 ℓ konzentrierte wässrige Ammoniak-Lösung gegossen und mehrmals mit Chloroform extrahiert. Die organischen Lösungen werden mit halbgesättigter Kochsalzlösung gewaschen, getrocknet und im Vakuum eingedampft. Der Rückstand wird in Chloroformlösung durch 159 g Kieselgel filtriert, mit 2 ℓ eines Gemisches (V/V) von Chloroform-Äthylacetat (9:1) nachgewaschen und eingedampft. Nach Umlösen aus Methylenchlorid-Äther erhält man 6,28 g 21-Chlor-6β-fluor-3β,5α,17α-trihydroxy-16-methylen-pregnan-20-on-3-acetat, Smp. 250-253° (Zers.).

In analoger Weise erhält man aus 5 g 21-Chlor-5α,6;16α,17-diepoxy-3β-hydroxy-16β-methyl-pregnan20-on-acetat und 100 ml der oben definierten Fluorwasserstoff-Harnstoff-Mischung nach der oben beschriebenen Verarbeitung und Chromatographie an 213 g Kieselgel [Elution mit einem Gemisch von Toluol-Äthylacetat (9 : 1)], 1,93 g des oben beschriebenen Endstoffes.

*Beispiel 7*

Ein Gemisch von 6,2 g 21-Chlor-6β-fluor-3β,5α,-17α-trihydroxy-16-methylen-pregnan-20-on-3-acetat und 40 ml Chloroform wird unter Rühren mit einer 45 Minuten vorher zubereiteten Mischungen von 96 ml Propionsäure und 80 m Trifluoressigsäureanhydrid versetzt und die gebildete Lösung während 17 Stunden im Dunkeln bei Zimmertemperatur stehen gelassen. Die dunkelrotbraune Reaktionslösung wird auf Eis geleert und unter Rühren innert 45 Minuten mit 200 g Natriumhydrogencarbonat versetzt. Nach weiteren 30 Minuten wird das Produkt in Methylenchlorid aufgenommen, mit gesättigter Natriumhydrogencarbonatlösung gewaschen, getrocknet und im Vakuum eingedampft. Der Rückstand in Toluollösung wird durch 160 g Kieselgel filtriert und mit 6 ℓ eines Gemisches (V/V) von Toluol-Äthylacetat (4 : 1) eluiert. Der Rückstand der im Vakuum eingedampften Eluate wird aus Äther-Pentan umkristallisiert, wobei man 6,8 g 21-Chlor-6β-fluor-3β,5α,-17α-trihydroxy-16-methylen-pregnan-20-on-3-acetat-5,17-dipropionat, Smp. 171-172°, erhält.

*Beispiel 8*

285 mg 21-Chlor-6β-fluor-3β,5α,17α-trihydroxy-16-methylen-pregnan-20-on-3-acetat-5,17-dipropionat, 20 mg Platinoxid und 25 ml Feinsprit werden in einer Wasserstoffatmosphäre bei Zimmertemperatur und einem Überdruck von 100-200 Torr bis zum Aufhören der Wasserstoffaufnahme gerührt. Der Katalysator wird abgenutscht und mit Methylenchlorid nachgewaschen, und das Filtrat im Vakuum eingedampft. Nach Umkristallisieren des Rückstandes aus Alkohol erhält man 21-Chlor-6β-fluor-3β,5α,17α-trihydroxy-16β-methyl-pregnan-20-on-3-acetat-5,17-dipropionat, Smp. 173 bis 174°.

*Beispiel 9*

Zu einer Mischung von 6,3 g 21-Chlor-6β-fluor-3β,5α,17α-trihydroxy-16β-methyl-pregnan-20-on-3-acetat-5,17-dipropionat und 315 ml Methanol gibt man unter Rühren 42 ml einer 2,85 N Lö-

sung von Chlorwasserstoff in Isopropylalkohol. Nach 3½ Stunden wird das Gemisch auf gesättigte Natriumhydrogencarbonatlösung geleert, der Niederschlag in Methylenchlorid aufgenommen, mit halbgesättigter Kochsalzlösung gewaschen, getrocknet und im Vakuum eingedampft. Das erhaltene 21-Chlor-6β-fluor-3β,5α,17α-trihydroxy-16β-methyl-pregnan-20-on-5,17-dipropionat wird in 42 ml Methylenchlorid und 168 ml Aceton gelöst und unter Rühren und Eiskühlung innert vier Minuten mit 6 ml einer 8 N wässrigen Lösung von Chromsäure in Schwefelsäure versetzt. Nach 30 Minuten versetzt man das Gemisch tropfenweise mit einer Lösung von 8,9 g Natriumacetat in 170 ml Wasser und extrahiert anschliessend mehrmals mit Methylenchlorid. Die organischen Auszüge werden mit gesättigter Natriumhydrogencarbonatlösung und mit Kochsalzlösung gewaschen, getrocknet und im Vakuum eingedampft. Das erhaltene 21-Chlor-6β-fluor-5α,17α-dihydroxy-16β-methyl-pregnan-3,20-dion-5,17-dipropionat schmilzt nach Umlösen aus Äther bei 145-147°.

*Beispiel 10*

In eine Lösung von 100 ml 21-Chlor-6β-fluor-5α,-17α-dihydroxy-16β-methyl-pregnan-3,20-dion-5,17-dipropionat in 10 ml Chloroform wird unter Rühren und Eiskühlung während 5 Stunden Chlorwasserstoffgas eingeleitet. Das Gemisch wird auf Eiswasser geleert und mit Methylenchlorid extrahiert, der Auszug mit 3%iger Lösung von Natriumhydrogencarbonat gewaschen, getrocknet und im Vakuum eingedampft. nach Umkristallisieren des Rückstandes aus Methylenchlorid-Äther erhält man 21-Chlor-6β-fluor-17α-hydroxy-16β-methyl-pregn-4-en-3,20-dion-propionat, Smp. 235,5-236°.

Zu derselben Verbindung gelangt man auch, wenn man das 21-Chlor-6β-fluor-5α,17α-dihydroxy-16β-methyl-pregnan-3,20-dion-5,17-dipropionat zunächst durch 9-stündiges Erhitzen mit Eisessig auf 80° in das 21-Chlor-6β-fluor-17α-hydroxy-16β-methyl-pregn-4-en-3,20-dion-propionat vom Smp. 174 bis 198° überführt und dieses anschliessend mit gasförmigem Chlorwasserstoff in Chloroform in der oben geschilderten Weise zum obigen Endstoff isomerisiert.

*Beispiel 11*

a) Zubereitung des hydroxylierenden Mikroorganismus-Präparates.

In einem 500 ml-Erlenmeyerkolben werden 100 ml einer sterilen Nährlösung (enthaltend 1 Gew.-% Hefeextrakt und 2 Gew.-% Saccharose, pH = 5,6) mit einer Schrägagar-Kultur von Curvularia lunata ATCC 12017 geimpft und 36 Stunden bei 28° und 120 U.p.M. geschüttelt. Mit 5 ml der erhaltenen Kulturlösung werden 100 ml einer sterilen Nährlösung [enthaltend 0,75% (Trockengewicht) «Corn-steep liquor» und 2 Gew.-% Saccharose, pH = 5,5] geimpft und unter den obigen Bedingungen 24 Stunden bebrütet. Das angewachsene Myzelium wird durch Zentrifugieren abgetrennt, in einem gleichen Volumen einer Lösung von 0,7 Gew.-% Natriumsulfat und 0,02 Gew.-% Tween 80® (Handelsname für Polysorbat 80) suspendiert, nochmals durch Zentrifugieren abgetrennt und in einer halben Menge der letztgenannten Lösung resuspendiert.

b) Transformation und Isolierung.

Mikrokristallines 21-Chlor-6α-fluor-17α-hydroxy-16β-methylpregn-4-en-3,20-dion-propionat wird in der oben beschriebenen Natriumsulfat-Tween 80-Lösung im Verhältnis 10 mg Steroid/1 ml Lösung suspendiert. Jeweils 40 Volumenteile der Myzelsuspension (siehe a) werden mit 1 Volumenteil der Steroidsuspension versetzt und in Erlenmeyerkolben während 48-96 Stunden bei 28° mit 250 U.p.M. geschüttelt. Die resultierende Kultursuspension wird mit Äthylacetat mehrmals extrahiert, der rohe Extrakt eingedampft und der Rückstand durch präparative Dünnschichtchromatographie [Silicagel; Gemisch von Toluol-Aceton (8 : 2)] getrennt. Neben einer Menge regeneriertem Ausgangsstoff wird als Umwandlungsprodukt nach Umlösen aus Äther das 21-Chlor-6α-fluor-11β,17α-dihydroxy-16β-methyl-pregn-4-en-3,20-dion-17-propionat erhalten; 360 MHz - NMR-Spektrum ($CDCl_3$): 1 (s) - $CH_3$ (18); 1,18 (t, J = 8,1 Hz) - $COCH_2\underline{CH_3}$; 1,39 (d, J = 8 Hz) - $CH_3$ (16); 1,44 (s) $CH_3$ (19); 3,95 + 4,05 (d, J = 12 Hz) - $\underline{CH_2}Cl$; 4,5 (m) - H (11); 5,1-5,4 (m) - H (6); 6,04 (s) - H (4) [ppm]; Smp. 214-214,5°C.

*Beispiel 12*

Ein Gemisch von 200 mg 21-Chlor-6α-fluor-11β,-17α-dihydroxy-16β-methyl-pregn-4-en-3,20-dion-17-propionat und 250 mg 2,3-Dichlor-5,6-dicyan-1,4-benzochinon in 8 ml Dioxan wird während 22,5 Stunden unter Rückfluss gekocht. Die abgekühlte Reaktionslösung wird mit 50 ml einer 5prozentigen (G/V) wässrigen Lösung von Natriumhydrogencarbonat versetzt, 30 Minuten gerührt und mit Methylenchlorid extrahiert. Die organische Phase wird mit verdünnter Kochsalzlösung gewaschen, getrocknet und im Vakuum eingedampft. Der Rückstand wird durch Dünnschichtchromatograohie auf Kieselgelplatten im System Toluol-Aceton (4 : 1) aufgetrennt. Das erhaltene 21-Chlor-6α-fluor-11β,17α-dihydroxy-16β-methyl-pregna-1,4-dien-3,20-dion-17-propionat wird aus Methylenchlorid-Methanol-Äther umgelöst, Smp. 221-222°C (Zersetzung).

*Beispiel 13*

In analoger Weise wie im Beispiel 12 wird 200 mg 21-Chlor-6α-fluor-17α-hydroxy-16β-methyl-pregn-4-en-3,20-dion-propionat (s. Beispiel 10) mit 250 mg 2,3-Dichlor-5,6-dicyan-1,4-benzochinon in 8 ml Dioxan behandelt und weiterverarbeitet, wodurch das 21-Chlor-6α-fluor-17α-hydroxy-16β-methyl-pregna-1,4-dien-3,20-dion-propionat resultiert; Smp. 216-219°C (aus Methylenchlorid-Äther).

*Beispiel 14*

Eine Salbe, enthaltend 0,1% 21-Chlor-6α-fluor-11β,17α-dihydroxy-16β-methyl-pregna-1,4-dien-3,20-dion-17-propionat, kann wie folgt hergestellt werden:

*Zusammensetzung* (in Gewichts-%)

| | |
|---|---|
| 21-Chlor-6α-fluor-11β,17α-dihydroxy-16β-methyl-pregna-1,4-dien-3,20-dion-17-proponat | 0,1 % |
| Vaseline | 45,0 % |
| Paraffinöl | 19,6 % |
| Cetylalkohol | 5,0 % |
| Bienenwachs | 5,0 % |
| Sorbitan-sesquioleat | 5,0 % |
| p-Hydroxybenzoesäureisopropylester | 0,2 % |
| Riechstoff | 0,1 % |
| Wasser | 20,0 % |

Die Fettstoffe und Emulgatoren werden zusammengeschmolzen. Das Konservierungsmittel wird in Wasser gelöst, und die Lösung in die Fettschmelze bei erhöhter Temperatur einemulgiert. nach dem Erkalten wird eine Suspension des Wirkstoffs in einem Teil der Fettschmelze in die Emulsion eingearbeitet und anschliessend Riechstoff zugegeben.

In analoger Weise wird auch eine Salbe, enthaltend 0,1% 21-Chlor-6α-fluor-11β,17α-dihydroxy-16β-methyl-pregn-4-en-3,20-dion-17-propionat bzw. 0,3% 21-Chlor-6α-fluor-17α-hydroxy-16β-methyl-pregn-4-en-3,20-dion-propionat oder dessen 1,2-Dehydroderivat — in letzteren 2 Fällen beträgt der Inhalt an Paraffinöl 19,4% —, hergestellt.

**Patentansprüche**

1. Verfahren zur Herstellung von halogenierten Steroiden der Formel

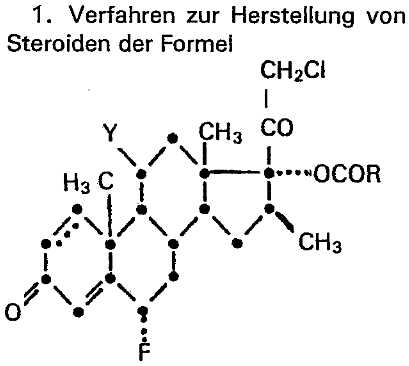

(A)

worin Y ein Wasserstoffatom oder Hydroxyl und R ein Alkyl mit höchstens 6 Kohlenstoffatomen bedeutet, wobei die punktierte Linie in der 1,2-Stellung die zusätzliche Doppelbindung eines 1,2-Dehydroderivats bezeichnet, dadurch gekennzeichnet, dass man nacheinander a) in das 3β-Hydroxy-16-methyl-pregna-5,16-dien-20-on oder ein 3-Carbonsäureester davon Chlor in die 21-Stellung einführt, b) das erhaltene 21-Chlor-3β-hydroxy-16-methyl-pregna-5,16-dien-20-on zu einem 3-Niederalkanoylester verestert und mit einer Persäure behandelt, c) in einem erhaltenen 5,6;16,17-Diepoxid den 16α,17α-Epoxidring unter Katalyse mit einer starken Säure zur 16-Methylen-17α-hydroxy-Gruppierung umlagert, den 5α,6α-Epoxidring mit Fluorwasserstoff in die 6β-Fluor-5α-hydroxy-Gruppierung überführt und die gebildete 5- und 17-Hydroxylgruppe verestert, d) den erhaltenen 21-Chlor-6β-fluor-3β,5α,17α-trihydroxy-16-methylen-pregnan-20-on-3-niederalkanoyl-5,17-diniederalkancarbonylester katalytisch hydriert, e) in der erhaltenen entsprechenden 16β-Methyl-Verbindung die veresterte 3-Hydroxylgruppe durch selektive Hydrolyse freisetzt und durch Behandlung mit einem Oxidationsmittel in die 3-Oxogruppe umwandelt, f) in einem erhaltenen 21-Chlor-6β-fluor-5α,17α-diniederalkancarbonyloxy-16β-methyl-pregnan-3,20-dion die 5-ständige Niederalkancarbonyloxygruppe sauer eliminiert und das 6β-Fluor unter Katalyse mit einer starken Säure in das 6α-Fluor-Isomere umwandelt und wenn eine Verbindung der Formel A, worin Y für Hydroxyl steht, erwünscht ist, ein erhaltenes Endprodukt der Formel A, worin Y für Wasserstoff steht, mittels des enzymatischen Systems eines 11β-hydroxylierenden Mikroorganismus in der 11β-Stellung hydrolysiert und/oder, wenn eine 1,2-Dehydroverbindung der Formel A erwünscht ist, ein erhaltenes 1,2-gesättigtes Endprodukt der Formel A dehydriert.

2. Verfahren gemäss Anspruch 1, dadurch gekennzeichnet, dass man zur Einführung des 21-Chloratoms gemäss Verfahrensstufe a) den Ausgangsstoff unter Katalyse mit einem Alkalimetallniederalkanolat mit einem Niederalkyloxalat oder -formiat zum entsprechenden 21-Niederalkoxalylderivat bzw. 21-Formylderivat umwandelt, aus diesem durch die Behandlung mit einem organischen Sulfonylazid das 21-Diazo-3β-hydroxy-16-methyl-pregna-5,16-dien-20-on bildet und dieses mit Chlorwasserstoff behandelt.

3. Verfahren gemäss Anspruch 1, dadurch gekennzeichnet, dass man 21-Chlor-3β-hydroxy-16-methyl-pregna-5,16-dien-20-on zur Veresterung der 3-Hydroxylgruppe mit einem symmetrischen Niederalkansäureanhydrid in 2,4,6-Collidin oder 2,6-Lutidin behandelt.

4. Verfahren gemäss Anspruch 1, dadurch gekennzeichnet, dass man zur Durchführung der Verfahrensstufe c) ein entsprechendens 5α,6;16α,17-Diepoxid mit Fluorwasserstoff behandelt.

5. Verfahren gemäss Anspruch 1, dadurch gekennzeichnet, dass man zur Durchführung der Verfahrensstufe c) ein entsprechendes 5β,6;16α,17-Diepoxid mit einer starken sauerstoffhaltigen Säure umsetzt, die erhaltene 5α,6β,17α-Trihydroxy-16-methylen-Verbindung mit einem organischen Sulfonylhalogenid in Gegenwart von 2,4,6-Collidin oder 2,6-Lutidin behandelt und die erhaltene 5α,6-Epoxy-17α-hydroxy-16-methylen-Verbindung mit Fluorwasserstoff umsetzt.

6. Verfahren gemäss Anspruch 1, dadurch gekennzeichnet, dass man zur Durchführung der Verfahrensstufe e) nacheinander mit einer starken Säure in einem oder mehreren Niederalkanolen und mit einer wässrigen Chromtrioxid-Schwefelsäure-Lösung in Aceton umsetzt.

7. Verfahren gemäss Anspruch 1, dadurch gekennzeichnet, dass man die biologische 11β-Hydroxylierung mit dem 11β-hydroxylierenden Enzymsystem von Curvularia lunata in wachsender oder ruhender Kultur durchführt.

8. Verfahren gemäss einem der Ansprüche 1-7, dadurch gekennzeichnet, dass man eine Verbindung der Formel A herstellt, die in der 1,2-Stellung gesättigt ist.

9. Ein halogeniertes Steroid der Formel

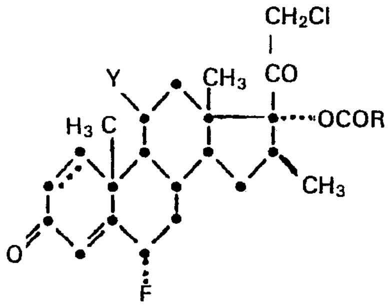

(A)

worin Y ein Wasserstoffatom oder Hydroxyl und R ein Alkyl mit höchstens 6 Kohlenstoffatomen bedeutet und die punktierte Linie in der 1,2-Stellung die zusätzliche Doppelbindung eines 1,2-Dehydroderivats bezeichnet.

10. Eine Verbindung der Formel A gemäss Anspruch 9, die in der 1,2-Stellung gesättigt ist.

11. 21-Chlor-6α-fluor-11β,17α-dihydroxy-16β-methyl-pregna-1,4-dien-3,20-dion-17-propionat.

12. 21-Chlor-6α-fluor-11β,17α-dihydroxy-16β-methyl-pregn-4-en-3,20-dion-17-propionat.

13. 21-Chlor-6α-fluor-17α-dihydroxy-16β-methyl-pregn-4-en-3,20-dion-17-propionat.

14. 21-Chlor-6α-fluor-17α-dihydroxy-16β-methyl-pregna-1,4-dien-3,20-dion-17-propionat.

15. Ein pharmazeutisches Präparat enthaltend eine der in den Ansprüchen 9-14 definierten Verbindungen zusammen mit einem pharmazeutischen Trägermaterial.

16. Eine Verbindung gemäss einem der Ansprüche 9-14 als Antiinflammatorikum.

**Claims**

1. Process for the manufacture of halogenated steroids of the formula

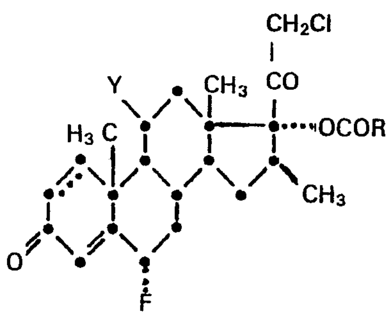

(A)

in which

Y represents a hydrogen atom or hydroxyl, and

R represents an alkyl radical having a maximum of 6 carbon atoms,

the dotted line in the 1,2-position representing the additional double bond of a 1,2-dehydro derivative, characterised in that, in succession,

a) chlorine is introduced into the 21-position of 3β-hydroxy-16-methyl-pregna-5,16-dien-20-one or a 3-carboxylic acid ester thereof,

b) the resulting 21-chloro-3β-hydroxy-16-methyl-pregna-5,16-dien-20-one is esterified to form a 3-lower alkanoyl ester and treated with a peracid,

c) in a resulting 5,6;16,17-diepoxide, the 16α,17α-epoxide ring is rearranged by catalysis with a strong acid to form the 16-methylene-17α-hydroxy grouping, the 5α,6α-epoxide ring is converted using hydrogen fluoride into the 6β-fluoro-5α-hydroxy-grouping and the 5- and 17-hydroxyl groups formed are esterified,

d) the resulting 21-chloro-6β-fluoro-3β,5α,17α-trihydroxy-16-methylene-pregnan-20-one 3-lower alkanoyl-5,17-di-lower alkanecarbonyl ester is catalytically hydrogenated,

e) in the resulting corresponding 16β-methyl compound, the esterified 3-hydroxyl group is freed by selective hydrolysis and converted into the 3-oxo group by treatment with an oxidising agent,

f) in a resulting 21-chloro-6β-fluoro-5α,17α-di-lower alkanecarbonyloxy-16β-methyl-pregnane-3,20-dione, the 5-positoned lower alkanecarbonyloxy group is removed by means of acid and the 6β-fluoro is converted by catalysis with a strong acid into the 6α-fluoro isomer,

and, if a compound of the formula A is desired in which Y represents hydroxyl, a resulting end produkt of the formula A in which Y represents hydrogen is hydroxylated in the 11β-position by means of the enzymatic system of a 11β-hydroxylating microorganism, and/or, if a 1,2-dehydro compound of the formula A is desiderd, a resulting 1,2-saturated end product of the formula A is dehydrogenated.

2. Process according to claim 1, characterised in that, for the introduction of the 21-chlorine atom according to process stage a), the starting material is converted, with catalysis by an alkali metal lower alkanolate, by a lower alkyl oxalate or formate into the corresponding 21-lower alkoxalyl derivative or 21-formyl derivative and 21-diazo-3β-hydroxy-16-methyl-pregna-5,16-dien-20-one is formed from this by treatment with an organic sulphonylazide and is then treated with hydrogen chloride.

3. Process according to claim 1, characterised in that 21-chloro-3β-hydroxy-16-methyl-pregna-5,16-dien-20-one is treated with a symmetrical lower alkanoic acid anhydride in 2,4,6-collidine or 2,6-lutidine in order to esterify the 3-hydroxyl group.

4. Process according to claim 1, characterised in that, in order to carry out process stage c), a corresponding 5α,6;16α,17-diepoxide is treated with hydrogen fluoride.

5. Process according to claim 1, characterised in that, in order to carry out process stage c), a corresponding 5β,6;16α,17-diepoxide is reacted with a strong oxygen-containing acid, the resulting 5α,6β,-17α-trihydroxy-16-methylene compound is treated with an organic sulphonyl halide in the presence of 2,4,6-collidine or 2,6 lutidine and the resulting 5α,6-epoxy-17α-hydroxy-16-methylene compound is reacted with hydrogen fluoride.

6. Process according to claim 1, characterised in that, in order to carry out process stage e), reaction is effected in succession with a strong acid in one or more lower alkanols, and with a solution of chromium trioxide in aqueous sulphuric acid, in acetone.

7. Process according to claim 1, characterised in that the biological 11β-hydroxylation is carried out with the 11β-hydroxylating enzyme systems of *Curvularia lunata* in a growing or static culture.

8. Process according to one of claims 1 to 7,

characterised in that a compound of the formula A is manufactured that is saturated in the 1,2-position.

9. A halogenated steroid of the formula

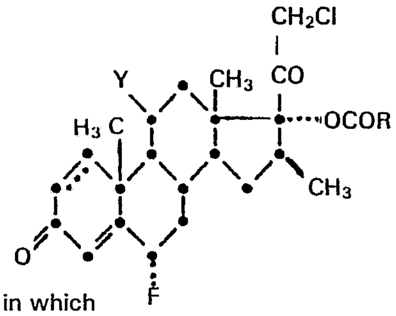

(A)

in which

Y represents a hydrogen atom or hydroxyl, and

R represents an alkyl radical having a maximum of 6 carbon atoms

and the dotted line in the 1,2-position represents the additional double bond of a 1,2-dehydro derivative.

10. A compound of the formula A according to claim 9 that is saturated in the 1,2-position.

11. 21-Chloro-6α-fluoro-11β,17α-dihydroxy-16β-methyl-pregna-1,4-diene-3,20-dione 17-propionate.

12. 21-Chloro-6α-fluoro-11β,17α-dihydroxy-16β-methyl-pregn-4-ene-3,20-dione 17-propionate.

13. 21-Chloro-6α-fluoro-17α-hydroxy-16β-methyl-pregn-4-ene-3,20-dione 17-propionate.

14. 21-Chloro-6α-fluoro-17α-hydroxy-16β-methyl-pregna-1,4-diene-3,20-dione 17-propionate.

15. A pharmaceutical preparation containing one of the compounds defined in claims 9 to 14 together with a pharmaceutical carrier.

16. A compound according to one of claims 9 to 14 as an anti-inflammatory agent.

**Revendications**

1. Procédé de préparation de stéroïdes halogénés de formule

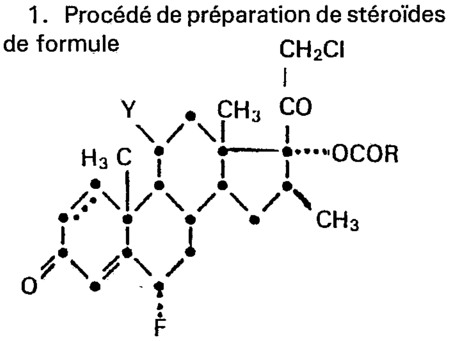

(A)

dans laquelle Y représente un atome d'hydrogène ou un groupe hydroxy et R un groupe alkyle à 6 atomes de carbone au maximum, le trait en pointillé en position 2 représentant la double liaison supplémentaire d'un dérivé 1,2-déshydrogéné, caractérisé en ce que, successivement, a) on introduit du chlore dans la position 21 de la 3 bêta-hydroxy-16-méthyl-prégna-5,16-diène-20-one ou un ester de ce composé et d'un acide carboxylique en position 3, b) on estérifie la 21-chloro-3-bêta-hydroxy-16-méthyl-prégna-5,16-diène-20-one obtenue en ester 3-alcanoylique inférieur et on traite par un peracide, c) dans un 5,6;16,17-diépoxyde obtenu, on transpose le cycle 16 alpha,17 alpha avec catalyse par un acide fort en le groupement 16-méthylène-17 alpha-hydroxy, on convertit le cycle 5 alpha, 6 alpha-époxyde par le fluorure d'hydrogène en le groupement 6 bêta-fluoro-5 alpha-hydroxy et on estérifie les groupes 5- et 17-hydroxy formés, d) on soumet l'ester 5,17-di-(alcane inférieur)-carbonylique, 3-alcanoulique inférieur de la 21-chloro-6 bêta-fluoro-3 bêta,5 alpha, 17 alpha-trihydroxy-16-méthylène- prégnane-20-one obtenu à hydrogénation catalytique, on libère du composé correspondant 16 bêta-méthylé ainsi obtenu le groupe 3-hydroxy estérifié par hydrolyse sélective et on le convertit en le groupe 3-oxo à l'aide d'un agent oxydant, f) on élimine de la 21-chloro-6 bêta-fluoro-5 alpha,17 alpha-di-(alcane inférieur)-carbonyloxy-16 bêta-méthyl-prégnane-3,20-dione obtenue le groupe (alcane inférieur)-carbonyle en position 5 à l'aide d'un acide et on convertit le substituant 6 bêta-fluoro par catalyse à l'aide d'un acide fort en l'isomère 6 alpha-fluoro et si l'on désire un composé de formule A dans laquelle Y représente le groupe hydroxy, on hydroxyle en position 11 bêta, à l'aide du système enzymatique d'un microorganisme hydroxylant en position 11 bêta, un produit final obtenu de formule A dans laquelle Y représente l'hydrogène, et/ou, si l'on veut un composé 1,2-déshydrogéné de formule A, on soumet un produit final 1,2-saturé obtenu, de formule A, à déshydrogénation.

2. Procédé selon la revendication 1, caractérisé en ce que, pour intraduire l'atome de chlore en position 21 dans le stade opératoire a), on convertit le produit de départ, avec catalyse à l'aide d'un alcanolate inférieur de métal alcalin, et à l'aide d'un oxalate ou formiate d'alkyle inférieur, en le dérivé 21-alcoxalylé inférieur correspondant ou le dérivé 21-formylé respectivement, à partir duquel, par traitement à l'aide d'un sulfonylazide organique, on forme la 21-diazo-3 bêta-hydroxy-16-méthyl-prégna-5,16-diène-20-one qu'on traite par le chlorure d'hydrogène.,

3. Procédé selon la revendication 1, caractérisé en ce que, pour estérifier le groupe 3-hydroxy de la 21-chloro-3 bêta-hydroxy-16-méthyl-prégna-5,16-diène-20-one, on traite par un anhydride symétrique d'acide alcanoïque inférieur dans la 2,4,6-collidine ou la 2,6-lutidine.

4. Procédé selon la revendication 1, caractérisé en ce que, pour la mise en œuvre du stade c), on traite un 5 alpha,6;16 alpha,17-diépoxyde correspondant par la fluorure d'hydrogène.

5. Procédé selon la revendication 1, caractérisé en ce que, pour la mise en œuvre du stade c), on fait réagir un 5 bêta,6;16 alpha,17-diépoxyde correspondant avec un acide oxygéné fort, ce qui donne un composé en 5 alpha, 6 bêta,17 alpha-trihydroxy-16-méthylène qu'on traite par un halogénure de sulfonyle organique en présence de 2,4,6-collidine ou de 2,6-lutidine, ce qui donne un composé en 5 alpha,6-époxy-17 alpha-hydroxy-16-méthylène qu'on fait réagir avec le fluorure d'hydrogène.

6. Procédé selon la revendication 1, caractérisé en ce que, pour la mise en œuvre du stade e), on fait réagir successivement avec un acide fort dans un ou plusieurs alcanols inférieurs et avec une solution

aqueuse de trioxyde de chrome-acide sulfurique dans l'acétone.

7. Procédé selon la revendication 1, caractérisé en ce que l'hydroxylation biologique en 11 bêta est effectuée à l'aide du système enzymatique hydroxylant en 11 bêta de Curvularia lunata en culture en prolifération ou en repos.

8. Procédé selon l'une quelconque des revendications 1 à 7, caractérisé en ce que l'on prépare un composé de formule A qui est saturé en position 1, 2.

9. Un stéroïde halogéné de formule

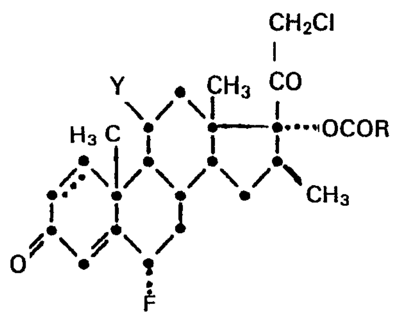

(A)

dans laquelle Y représente un atome d'hydrogène ou un groupe hydroxy et R représente un groupe alkyle à 6 atomes de carbone au maximum, le trait en pointillé en position 1, 2 représentant la double liaison supplémentaire d'un dérivé 1,2-déshydrogéné.

10. Un composé de formule A selon la revendications 9, saturé en position 1, 2.

11. Le 17-propionate de la 21-chloro-6 alpha-fluoro-11 bêta,17 alpha-dihydroxy-16 bêta-méthyl-prégna-1,4-diène-3,20-dione.

12. Le 17-propionate de la 21-chloro-6 alpha-fluoro-11 bêta,17 alpha-dihydroxy-16 bêta-méthyl-prégna-4-ène-3,20-dione.

13. Le 17-propionate de la 21-chloro-6 alpha-fluoro-17 alpha-hydroxy-16 bêta-méthyl-prégna-4-ène-3,20-dione.

14. Le 17-propionate de la 21-chloro-6 alpha-fluoro-17 alpha-dihydroxy-16 bêta-méthyl-prégna-1,4-diène-3,20-dione.

15. Une composition pharmaceutique contenant l'un des composés définis dans les revendications 9 à 14, avec un véhicule pharmaceutique.

16. Un composé selon l'une des revendications 9 à 14, en tant qu'agent anti-inflammatoire.